# EUROPÄISCHE PATENTSCHRIFT

(11) **EP 1 230 206 B1**
(45) Veröffentlichungstag und Bekanntmachung des Hinweises auf die Patenterteilung: **25.02.2004**
(21) Anmeldenummer: 00983116.5
(22) Anmeldetag: 08.11.2000
(51) Int. Cl.: C07C 51/48, C07C 51/09, C07C 51/44, C07C 53/02

(54) **VERFAHREN ZUR HERSTELLUNG VON AMEISENSÄURE**
METHOD FOR PRODUCTION OF FORMIC ACID
PROCEDE DE PRODUCTION D'ACIDE FORMIQUE

(30) Priorität: 09.11.1999 DE 19953832
(43) Veröffentlichungstag der Anmeldung: 14.08.2002
(73) Patentinhaber: BASF AKTIENGESELLSCHAFT, 67056 Ludwigshafen (DE)
(72) Erfinder: HLADIY, Serhiy, Boryslav, 293760 (UA); STARCHEVSKYY, Mykhaylo, Boryslav, 293760 (UA); PAZDERSKYY, Yuriy, Boryslav, 293760 (UA); LASTOVYAK, Yaroslav, Drohobych, 293720 (UA)
(74) Vertreter: Isenbruck, Günter, Dr.
(86) Internationale Anmeldenummer: PCT/EP2000/011046
(87) Internationale Veröffentlichungsnummer: WO 2001/034545

(56) Entgegenhaltungen:
- GB-A- 371 554
- US-A- 4 326 073

## Beschreibung

Die Erfindung betrifft ein Verfahren zur Herstellung von Ameisensäure.

Neuere Verfahren zur Herstellung von Ameisensäure im technischen Maßstab gehen von Methylformiat aus, das durch Carbonylierung von Methanol einfach zugänglich ist. Das Methylformiat wird anschließend hydrolisiert, wobei die Ameisensäure als Katalysator wirkt. Da sowohl die Verseifung wie auch die Rückveresterung katalysiert wird, stellt sich ein Gleichgewicht ein, in welchem alle vier Komponenten, Methylformiat, Wasser, Ameisensäure und Methanol in hohen Anteilen vorliegen.

Dies führt zu Schwierigkeiten bei der Reaktionsführung. Eine Gleichgewichtsverschiebung durch destillative Entfernung der gewünschten Verfahrensprodukte ist nicht möglich, weil das Methylformiat (Siedepunkt 32°C) wesentlich tiefer siedet als Methanol (Siedepunkt 65°C) und Ameisensäure (Siedepunkt 101°C). Günstig wäre es daher, das Gleichgewicht durch einen Überschuß an Wasser auf die Seite der Ameisensäure zu verschieben.

Weitere Schwierigkeiten treten bei der Aufarbeitung der wässrigen Ameisensäure auf. Ameisensäure und Wasser bilden ein Aceotrop, das 77,5 Gew.-% HCOOH enthält und bei 101,3 kPa bei 107,1°C siedet. Die bei der Hydrolyse von Methylformiat anfallende wässrige Ameisensäure besitzt einen Säuregehalt von ca. 20 bis 60 Gew.-%. Aus diesen verdünnten wässrigen Ameisensäurelösungen lassen sich daher nicht ohne weiteres reine oder höher konzentrierte Ameisensäure destillativ gewinnen.

In der US 2,160,064 wird vorgeschlagen, das Aceotrop durch Destillation bei verschiedenen Drücken aufzutrennen. Dazu wird die verdünnte Säure zunächst bei relativ hohem Druck in Wasser als Kopfprodukt und ein an Ameisensäure reiches Aceotrop als Sumpfprodukt aufgetrennt. Dieses Aceotrop wird anschließend in einer zweiten, bei relativ geringerem Druck betriebenen Kolonne erneut destilliert. Dabei erhält man als Kopfprodukt Ameisensäure und als Sumpfprodukt ein Aceotrop, mit einem geringeren Säuregehalt als das der ersten Destillationsstufe. Das Aceotrop der zweiten Stufe wird wieder zur ersten Stufe zurückgeführt.

Bei einer praktischen Ausführung wird die Destillation bei einem Druck von ungefähr 202,6 bis 303,9 kPa ausgeführt. Dadurch wird die Zusammensetzung des Aceotrops hin zu einem höheren Säuregehalt verschoben. Theoretisch sollte das Aceotrop bei 253,2 kPa ungefähr 84 bis 85 Gew.-% Ameisensäure enthalten. Bei der praktischen Durchführung der Destillation übersteigt der Gehalt an Ameisensäure im Sumpf der ersten Destillationskolonne nie einen Anteil von 81 bis 82 Gew.-%. Die zweite Destillation wird bei atmosphärischem Druck oder etwas unterhalb des Atmosphärendrucks durchgeführt. Dadurch kann die Menge an Ameisensäure abdestilliert werden, die dem Unterschied in der Zusammensetzung der beiden Aceotrope entspricht. Wegen der geringen Unterschiede müssen die Kolonnen eine sehr hohe theoretische Bodenzahl aufweisen und mit sehr hohen Rückflußverhältnissen betrieben werden. Bei einem Rückflußverhältnis von R = 2,3 beträgt die Zahl der theoretischen Böden für die erste Destillationskolonne 15. Die zweite Kolonne weist bei einem Rückflußverhältnis von R = 10 eine theoretische Bodenzahl von 18 auf. Weiter muß das bei der zweiten Destillation als Sumpf anfallende Aceotrop wieder zur ersten Destillationsstufe zurückgeführt werden.

Sowohl wegen der hohen Sumpftemperatur (125 bis 135°C) in der ersten Kolonne, wie auch wegen der hohen Verweilzeit der Ameisensäure sind hohe Ausbeuteverluste durch die Zersetzung der Ameisensäure hinzunehmen. Ferner müssen wegen der Aggressivität von wässriger Ameisensäure Destillationskolonnen aus speziellen Werkstoffen eingesetzt werden, um übermäßige Korrosion zu vermeiden. Wegen der großen im Kreislauf geführten Produktströme und dem hohen Energieverbrauch ist das beschriebene Verfahren daher für einen Einsatz im technischen Maßstab ungünstig.

Man ist daher dazu übergegangen, die Ameisensäure aus dem Gemisch mit Wasser extraktiv abzutrennen, wobei Extraktionsmittel und Ameisensäure in einem weiteren Schritt destillativ getrennt werden. Ein solches Verfahren wird beispielsweise in der EP 0 017 866 B1 vorgeschlagen. Dabei wird zunächst Methylformiat hydrolisiert und aus dem so erhaltenen Hydrolysegemisch das Methanol sowie überschüssiges Methylformiat abdestilliert. Das aus Ameisensäure und Wasser bestehende Sumpfprodukt der Destillation wird in einer Flüssigextraktion mit einem hauptsächlich die Ameisensäure aufnehmenden Extraktionsmittel extrahiert. Als bevorzugte Extraktionsmittel werden Carbonsäureamide vorgeschlagen, insbesondere N-Di-n-butylformamid, N-Di-n-butylacetamid, N-Methyl-N-2-heptylformamid, N-n-Butyl-N-2-ethylhexyl-formamid, N-n-Butyl-N-cyclohexylformamid, N-Ethylformanilid sowie Gemische dieser Verbindungen. Als weitere geeignete Extraktionsmittel sind u.a. Isopropylether, Methylisobutylketon, Ethylacetat, Tributylphosphat und Butandiolformiat beschrieben. Das bei der Extraktion erhaltene Gemisch aus Ameisensäure, dem Extraktionsmittel und einem Teil des Wassers wird einer weiteren Destillation unterworfen. Am Kopf der Kolonne wird ein Produkt, das aus der Gesamtmenge oder einer Teilmenge des in die Destillation eingeführten Wassers und einem Teil der Ameisensäure besteht, abgenommen und dampfförmig in den unteren Teil der ersten Destillationskolonne zurückgeführt. Im Sumpf fällt ein Gemisch aus Extraktionsmittel, gegebenenfalls einer Teilmenge des Wassers und dem Großteil der Ameisensäure an. Dieses Gemisch wird in einer weiteren, dritten Destillationsstufe in wasserfreie bzw. weitgehend wasserfreie Ameisensäure und das Extraktionsmittel aufgetrennt. Das Extraktionsmittel wird wieder in das Verfahren zurückgeführt. In einer besonderen Ausfühmngsform sind die erste und die zweite Destillationsstufe in einer gemeinsamen Kolonne zusammengefaßt. Die Extraktion der Ameisensäure aus dem wässrigen Gemisch erfolgt jedoch weiter in einem getrennten Extraktor. Dazu wird der Kolonne über einen Seitenabzug das Gemisch aus Ameisensäure und Wasser entnommen und dem Extraktor zugeleitet. Das Gemisch aus Extraktionsmittel und Ameisensäure und gegebenenfalls Wasser wird aus dem Extraktor ausgeleitet und unterhalb des Seitenabzugs der Destillationskolonne wieder zugeführt. Im Sumpf der Kolonne wird ein Gemisch aus Ameisensäure, Extraktionsmittel und gegebenenfalls Wasser abgenommen und einer weiteren, Destillationskolonne zugeführt, die bei der oben beschriebenen Ausführungsform der dritten Destillationsstufe entspricht.

Die Gestaltung großtechnischer Synthesen wird stark von wirtschaftlichen Überlegungen bestimmt. Aufgabe der Erfindung ist daher, ein Verfahren zur Gewinnung von Ameisensäure zur Verfügung zu stellen, das günstige, großtechnisch verfügbare Chemikalien einsetzt, die nicht umweltschädlich sein sollten, und das im Vergleich zu den aus dem Stand der Technik bekannten Verfahren in kleineren Anlagen bei gleicher Ausbeute durchzuführen ist.

Diese Aufgabe wird gelöst durch ein Verfahren zur Herstellung von Ameisensäure, umfassend die Schritte:
a) Hydrolyse von Methylformiat unter Erhalt eines Gemisches aus Wasser, Ameisensäure, Methanol und überschüssigem Methylformiat;
b) destillative Abtrennung des Methanols und des überschüssigen Methylformiats aus dem Gemisch aus Wasser, Ameisensäure, Methanol und überschüssigem Methylformiat unter Erhalt von wässriger Ameisensäure;
c) Extraktion der wässrigen Ameisensäure mit mindestens einem Ameisensäureester aus der Gruppe, die gebildet ist von Ethylenglycoldiformiat, Diethylenglycoldiformiat, 1,2-Propandioldiformiat, 2,3-Propandioldiformiat, Dipropylenglycoldiformiat, 2,3-Butandioldiformiat, 1,4-Butandioldiformiat, Benzylforrniat, Cyclohexylformiat, 2-Phenylformiat, 2-Ethylhexylformiat unter Erhalt eines Gemisches aus Ameisensäureester und Ameisensäure unter Destillationsbedingungen in einer Extraktivdestillationskolonne;
d) destillative Trennung von Ameisensäureester und Ameisensäure.

Die genannten Ameisensäureester werden großtechnisch hergestellt und stehen damit in großen Mengen bei günstigen Kosten zur Verfugung. Besonders bevorzugt ist Benzylformiat.

Benzylformiat ist eine farblose Flüssigkeit mit einem leichten Zimtgeruch. Sie weist eine Dichte von 1,04 g pro cm³ und einen Siedepunkt von 202,3°C auf. Mit Wasser bildet Benzylformiat ein Aceotrop mit einem Wassergehalt von 80 Gew.-% und einem Siedepunkt von 99,2°C. Benzylformiat weist als Extraktionsmittel ideale Eigenschaften auf Der Siedepunkt liegt hoch genug, um eine wirksame und einfache destillative Abtrennung der Ameisensäure zu erlauben. Gleichzeitig ist bei den erforderlichen Temperaturen noch keine merkliche Zersetzung der Ameisensäure zu beobachten. Bei dem erfindungsgemäßen Verfahren ist daher die Ameisensäure nur sehr geringen thermischen Belastungen ausgesetzt, so dass nur eine geringe Zersetzung der Ameisensäure in Kauf genommen werden muss. Ferner liegt die Ameisensäure sowohl in der wässrigen Lösung wie auch im Extraktionsmittel in vergleichsweise niedriger Konzentration vor. Zusammen mit den niedrigen Temperaturen, die im erfindungsgemäßen Verfahren erforderlich sind, führt dies zu einer nur geringen Korrosionswirkung der Ameisensäure. Bis auf die als letzte Stufe vorgesehene destillative Trennung von Ameisensäure und Extraktionsmittel können daher für den Bau der Anlage weniger widerstandsfähige und damit kostengünstigere Materialien verwendet werden. Benzylformiat ist eine bei den Reaktionsbedingungen des Verfahrens stabile Verbindung. Bei der Aufarbeitung müssen daher keine Zersetzungsprodukte des Extraktionsmittels abgetrennt werden. Ferner zeigt Benzylformiat keine Wechselwirkung mit den beim Verfahren verwendeten Substanzen. Als weiterer Vorteil ist die fehlende Giftigkeit von Benzylformiat anzuführen. Solche Ester kommen natürlich als Aromastoffe in verschiedenen Früchten in größeren Mengen vor. Sie werden weiter in Parfums sowie als Aromastoffe in Lebensmitteln verwendet. Schließlich wird Benzylformiat aus Benzylalkohol hergestellt, das als Großchemikalie in praktisch unbegrenzter Menge bei niedrigen Preisen zur Verfügung steht.

Die für Benzylformiat angeführten Vorteile gelten natürlich auch für die anderen genannten Ameisensäureester mehr oder weniger stark ausgeprägt.

Die Extraktion erfolgt unter Destillationsbedingungen in einer extraktiven Destillationskolonne. Es entfällt dadurch die Notwendigkeit eines separaten Extraktors. Am Kopf der Kolonne wird ein Gemisch aus dem größten Teil des Wassers, wenig Ameisensäure und geringen Mengen des als Extraktionsmittel wirkenden Ameisensäureesters abgenommen. Dieses Gemisch wird zum Hydrolysereaktor, in dem eine Verseifung des Methylformiats erfolgt, zurückgeführt. Am Sumpf wird ein Gemisch abgenommen, das aus geringen Mengen Wasser, dem größten Teil der Ameisensäure und dem größten Teil des Extraktionsmittels besteht.

Der Ameisensäureester muß nicht als Substanz eingeleitet werden. Vorteilhaft ist es auch möglich, den entsprechenden Alkohol einzusetzen. Dieser setzt sich unter den Bedingungen der Extraktivdestillation "in situ" mit der Ameisensäure zum Ameisensäureester um. Durch das Reaktionsgleichgewicht zwischen Ameisensäureester und Alkohol sind im Reaktionsgemisch des Verfahrens immer geringe Mengen an Alkohol vorhanden. Diese sind jedoch insbesondere im Fall des Benzylalkohols nicht weiter störend, da Benzylalkohol vergleichbare physikalische Eigenschaften aufweist wie Benzylformiat. Benzylalkohol weist eine Dichte von 1,04 g pro cm³ und einen Siedepunkt von 205,3°C auf. Mit Wasser bildet es ein Aceotrop mit einem Gehalt von 91 Gew.-% H₂O, das bei 99,9°C siedet. In der praktischen Ausführung des Verfahrens kann daher das Gemisch aus Benzylformiat und Benzylalkohol wegen der vergleichbaren physikalischen Eigenschaften und der raschen Umsetzung zwischen den beiden Substanzen als eine Substanz angesehen werden.

Der Ameisensäureester und/oder der Alkohol wird günstig am Kopf der Extraktivdestillationskolonne aufgegeben.

Die Extraktivdestillationskolonne wird vorteilhaft bei Umgebungsdruck betrieben.

Am Kolonnenkopf der Extraktivdestillationskolonne ist bei einer vorteilhaften Ausfiihrungsform ein Phasenscheider vorgesehen. Das am Kolonnenkopf abgenommene Gemisch aus Wasser, wenig Ameisensäure und etwas Extraktionsmittel kann dann dem Separator zugeführt werden. Dort können sich die wässrige Phase und die Phase des Extraktionsmittels trennen und das Extraktionsmittel anschließend der Extraktivdestillationskolonne wieder zugeführt werden.

Das im Sumpf der Extraktivdestillationskolonne abgenommene Gemisch aus Ameisensäure, Ameisensäureester und gegebenenfalls geringen Mengen an Wasser wird destillativ aufgearbeitet. Dabei hat es sich als günstig erwiesen, daß die Auftrennung des Gemisches aus Ameisensäureester und Ameisensäure bei vermindertertem Druck in einer Vakuumkolonne erfolgt. Dadurch erniedrigt sich die thermische Belastung der Ameisensäure, wodurch Verluste durch Zersetzung weiter reduziert werden. Ferner wirkt sich die geringere Destillationstemperatur vorteilhaft auf die Energiebilanz und damit die Kosten des Verfahrens aus.

Der Ameisensäureester kann zwischen Extraktivdestillationskolonne und Vakuumkolonne im Kreislauf geführt werden. Dadurch müssen nur geringe, unvermeidliche Verluste an Extraktionsmittel ergänzt werden.

Der Wasseranteil im Sumpfgemisch der Extraktivdestillationskolonne wird durch den Anteil des Ameisensäureesters, beispielsweise Benzylformiat, bestimmt. Mit dem erfindungsgemäßen Verfahren kann daher sowohl wasserfreie Ameisensäure als auch wässrige Ameisensäure mit einem bestimmten Wasseranteil hergestellt werden. Vorteilhaft wird daher die Menge des Ameisensäureesters in Abhängigkeit vom Wassergehalt der zu gewinnenden Ameisensäure gewählt.

Vorteilhaft wird die Extraktivdestillationskolonne bei minimalem Rückflußverhältnis betrieben.

Die Erfindung wird im weiteren anhand von Beispielen sowie unter Bezugnahme auf eine Zeichnung näher erläutert. Es zeigt:

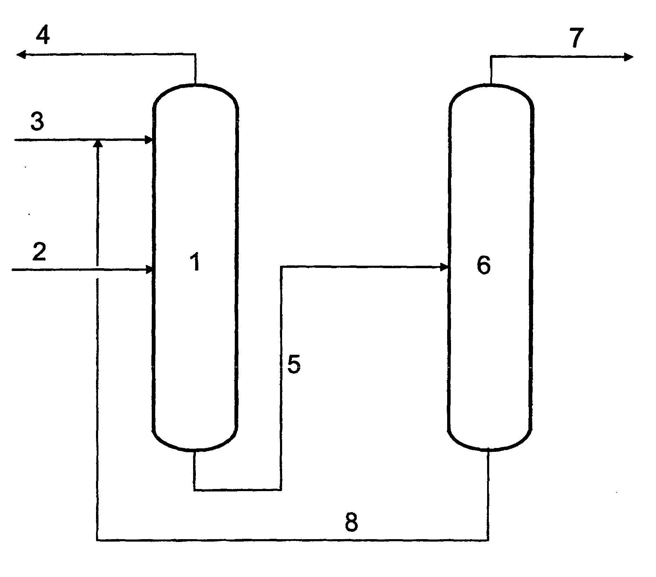

- Fig. 1: eine schematische Darstellung einer Vorrichtung zur Durchführung des erfindungsgemäßen Verfahrens;

In Fig. 1 bezeichnet 1 eine Extraktivdestillationskolonne. Dieser wird über Leitung 2 aus dem (nicht dargestellten) Hydrolysereaktor nach Abtrennung von Methanol und überschüssigem Methylformiat ein Gemisch aus Ameisensäure und Wasser zugeführt. Das Gemisch kann bei entsprechender Reaktionsführung auch noch geringe Mengen des Extraktionsmittels Benzylformiat enthalten. Die Zuleitung des Hydrolysegemisches erfolgt etwa in der Mitte der Extraktivdestillationskolonne. Die Zuführung des Extraktionsmittels Benzylformiat erfolgt über Zuleitung 3 nahe dem Kopf der Kolonne. Während des kontinuierlichen Betriebs der Anlage müssen nur die unvermeidlichen Verluste an Extraktionsmittel ausgeglichen werden. Der größte Anteil des Extraktionsmittels wird im Kreislauf geführt. Am Kopf der Extraktivdestillationskolonne wird über Ableitung 4 ein Gemisch abgenommen, das den größten Teil des Wassers sowie geringe Mengen an Ameisensäure und Extraktionsmittel enthält. Das Extraktionsmittel kann gegebenenfalls in einem (nicht dargestellten) Phasenscheider abgetrennt werden. Das Gemisch wird in den Hydrolysereaktor (nicht dargestellt) zurückgeführt. Am Sumpf der Extraktivdestillationskolonne 1 wird über Leitung 5 ein Gemisch abgeführt, das den überwiegenden Anteil der Ameisensäure und des Extraktionsmittels sowie gegebenenfalls geringe Mengen Wasser umfaßt. Leitung 5 mündet in einer Vakuumkolonne 6, wobei die Zuleitung in etwa der Mitte der Höhe der Kolonne erfolgt. Am Kopf der Kolonne wird über Ableitung 7 Ameisensäure abgenommen, die gegebenenfalls geringe Mengen an Wasser enthalten kann. Am Sumpf der Kolonne wird das Extraktionsmittel abgenommen, das nahezu frei von Ameisensäure und Wasser ist und über Umlaufleitung 8 und Zuleitung 3 wieder der Extraktivdestillationskolonne 1 zugeführt.

Bei den angegebenen Bedingungen wurde eine Zersetzung des Extraktionsmittels nicht beobachtet.

Die Bedingungen für die Durchführung der extraktiven Destillation sowie die Auftrennung des Gemisches aus Benzylformiat und Ameisensäure werden anhand der folgenden Beispiele genauer erläutert.

### 1. Destillation

### 1.1 Destillationskolonne

Eine Glassäule mit einem Innendurchmesser von 20 mm wurde mit Glasringen (4 mm) bis zu einer Höhe von 1,470 m gepackt. Die Höhe eines theoretischen Bodens wurde mit einer Benzol-Dichlormethan-Standardmischung zu 84 mm bestimmt. Der Zustrom wurde in Höhe des 11. theoretischen Boden eingeleitet. Das Extraktionsmittel wurde am Kopf der Kolonne aufgegeben. Am Kopf der Kolonne war ein Phasenscheider angebracht, in dem das abdestillierte Gemisch in Extraktionsmittel und wässriger Ameisensäure aufgetrennt wurde. Die Kapazität des Sumpfes der Kolonne betrug 500 cm³.

### 1.2 Verfahrensbedingungen für die Extraktivdestillation

Die Kolonne wurde bei Atmosphärendruck betrieben. Es wurden Zusammensetzungen mit unterschiedlichem Wasser- bzw. Ameisensäuregehalt eingeleitet und nach Einstellung der Prozeßparameter die Zusammensetzung des am Kopf der Kolonne abgenommenen Destillats sowie des im Sumpf abgenommenen Extraktes untersucht. Die Verfahrensbedingungen der Kolonne waren wie folgt:

| Ort | Zuleitung | Kopf | Sumpf |
|---|---|---|---|
| Temperatur, C° | 101 bis 105 | 98 bis 99 | 115 bis 145 |

Das am Kopf der Kolonne abgenommene Destillat enthielt bei 99°C 98,8 Gew.-% H₂O, 0,6 Gew.-% Ameisensäure und 0,6 Gew.-% Benzylformiat.

Die für verschiedene Molverhältnisse von Benzylformiat und Ameisensäure erhaltenen Ergebnisse sind in Tabelle 1 aufgeführt.

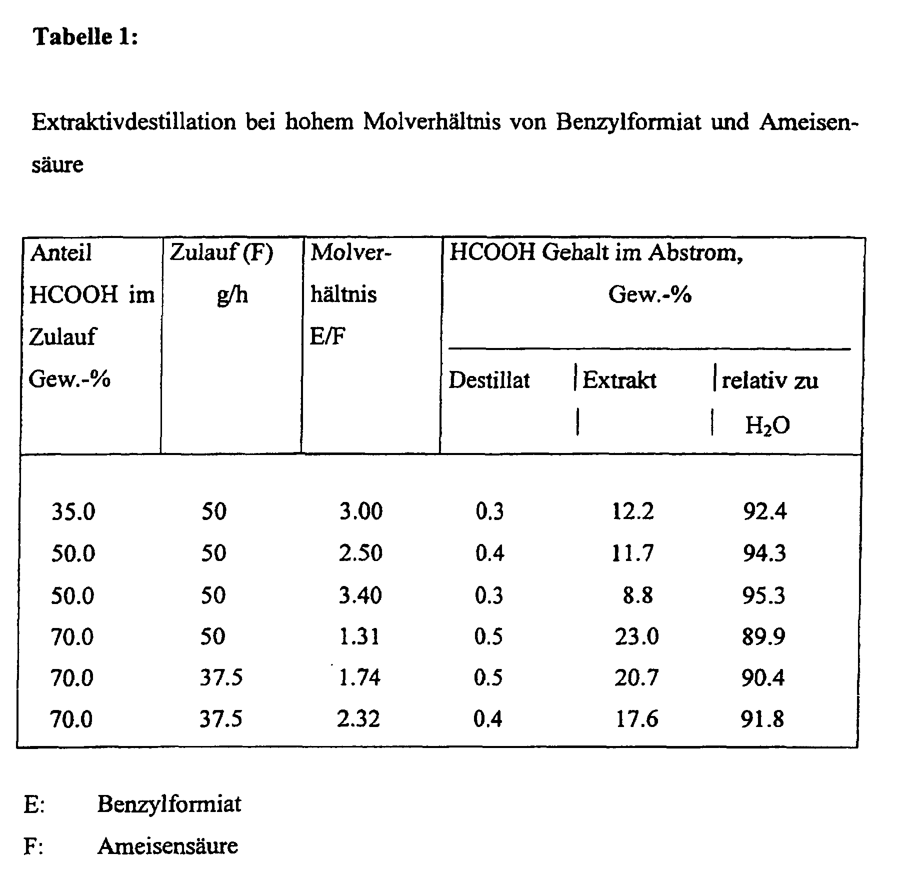

### 1.3 Vakuumdestillation des Gemisches aus Ameisensäure und Benzylformiat

Die Destillation wurde mit derselben Kolonne ausgeführt wie unter 1.1 beschrieben. Die Destillation wurde bei einem Druck von 13,3 bis 14,7 kPa durchgeführt. Die Temperaturen in der Kolonne waren wie folgt:

| Ort | Zulauf | Kopf | Sumpf |
|---|---|---|---|
| Temperatur, °C | 101 bis 105 | 50 bis 52 | 143 bis 145 |

Die bei der Destillation erhaltenen Daten sind in Tabelle 2 zusammengefaßt.

**Tabelle 2:**

| Destillation von Ameisensäure aus einem Extrakt | | | |
|---|---|---|---|
| Strom | Gehalt Gew.-% | | |
| | HCOOH | H₂O | Benzylformiat |
| Zustrom | 9.0 | 0.4 | 90.6 |
| Destillat | 94.3 | 5.7 | ∼ 0.01 |
| Sumpf | 0.3 | ∼ 0.01 | 99.7 |

### 2. Abhängigkeit des Flüssig-/Dampfförmig-Gleichgewichtes von der H₂O-HCOOH-Benzylformiat-Zusammensetzung

In einem 500 ml Kolben mit Rückflußkühler und Destillationsbrücke wurde jeweils eine definierte Mischung aus Wasser, Ameisensäure und Benzylformiat vorgelegt und zum Sieden erhitzt. Nach Einstellung des Gleichgewichts wurde jeweils 1 ml des Destillats abgenommen und auf seine Zusammensetzung hin untersucht. Die Zusammensetzung des Gemisches in der Vorlage wurde jeweils so variiert, daß von einem jeweils konstantem Anteil von Benzylformiat ausgegangen wurde und das Verhältnis von Wasser und Ameisensäure variiert wurde.

In einer ersten Versuchsreihe wurde der Anteil des Benzylformiats zu 30 Mol-% gewählt. In Tabelle 3 sind jeweils der Wasseranteil in der flüssigen (X) und der dampfförmigen (Y) Phase relativ zur Ameisensäure angegeben.

**Tabelle 3**

| Gehalt an H₂O (Mol-%) in flüssiger und dampfförmiger Phase (30 Mol-% Benzylformiat) | | | | | | | | | | |
|---|---|---|---|---|---|---|---|---|---|---|
| X | 9.31 | 18.41 | 24.52 | 33.20 | 41.59 | 50.05 | 61.71 | 69.90 | 83.75 | 92.50 |
| Y | 4.53 | 19.52 | 32.27 | 42.93 | 58.46 | 63.03 | 73.85 | 82.45 | 92.55 | 97.12 |

Bei einem Anteil an Benzylformiat von 30 Mol-% beträgt die Zusammensetzung des Aceotrops aus H₂O und Ameisensäure 17.5 Mol-% H₂O.

In einer zweiten Versuchsreihe wurde der Anteil des Benzylformiats auf 45.0 Mol-% eingestellt. Der Wasseranteil in der flüssigen bzw. dampfförmigen Phase ist in Tabelle 4 angegeben.

**Tabelle 4**

| Gehalt an H₂O (Mol-%) in flüssiger und dampfförmiger Phase (45 Mol-% Benzylformiat) | | | | | | | |
|---|---|---|---|---|---|---|---|
| X | 2.11 | 5.16 | 7.36 | 12.87 | 16.99 | 25.35 | 27.12 |
| Y | 2.66 | 6.73 | 10.62 | 16.30 | 22.97 | 39.90 | 36.56 |
| X | 31.82 | 40.18 | 46.63 | 51.30 | 70.80 | 88.44 | 94.50 |
| Y | 45.56 | 58.42 | 64.71 | 68.12 | 85.22 | 96.35 | 38.08 |

Bei einem Anteil von 45 Mol-% Benzylformiat bildet die Mischung aus H₂O und Ameisensäure kein Aceotrop mehr. Aus den Gleichgewichtsdaten kann das mindestens erforderliche Rückflußverhältnis sowie die Anzahl der theoretischen Böden für die Extraktivdestillationskolonne ermittelt werden. In diesem Fall beträgt das mindestens erforderliche Rückflußverhältnis aus Rₘᵢₙ = 0.6. Die Anzahl der theoretischen Böden beträgt 9 für den unteren Teil und 4 für den oberen Teil der Extraktivdestillationskolonne.

### 3. Gleichgewichtsverteilung im System Wasser-Ameisensäure-Benzylformiat in der flüssigen Phase

Ein Gemisch aus Wasser (10.0061 g) und Benzylformiat (E, 10,7949 g) wurden jeweils in ein thermostatisiertes Glasgefäß gegeben und bei 20,0 (+- 0,2) °C gerührt. Dieser Mischung wurde jeweils eine geringe Menge an Ameisensäure zu-gegeben. Nach intensiven Mischen wurde der Rührer angehalten und bis zur Phasentrennung abgewartet. Es wurden jeweils Proben aus der Wasserphase und der Benzylformiatphase entnommen und auf ihren Ameisensäuregehalt untersucht. Die ermittelten Werte sind in Tabelle 5 angegeben.

**Tabelle 5**

| Verteilung von Ameisensäure zwischen Wasser und Benzylformiat bei 20°C | | | | | | | | |
|---|---|---|---|---|---|---|---|---|
| HCCOH g | Benzylformiat Gew.-% | | | | Wasser, Gew.-% | | | |
| | E | HCOOH | H₂O | BA | H₂O | HCOOH | E | BA |
| 0 | 92.9 | 0 | 1.5 | 5.6 | 99.1 | 0 | 0.5 | 0.4 |
| 0.5861 | 88.4 | 0.8 | 2.1 | 8.7 | 89.0 | 9.8 | 0.7 | 0.5 |
| 1.5912 | 86.9 | 2.1 | 2.3 | 8.7 | 82.9 | 15.2 | 1.2 | 0.7 |
| 2.1897 | 84.2 | 3.0 | 2.7 | 10.1 | 79.2 | 18.1 | 1.5 | 1.2 |
| 3.2370 | 79.9 | 4.7 | 3.3 | 12.1 | 73.4 | 23.3 | 1.8 | 1.5 |
| 4.3963 | 73.9 | 7.1 | 4.7 | 14.3 | 65.8 | 30.3 | 2.1 | 1.8 |
| E: Benzylformiat BA: Benzylalkohol | | | | | | | | |

## Patentansprüche

1. Verfahren zur Herstellung von Ameisensäure umfassend die Schritte:
a) Hydrolyse von Methylformiat unter Erhalt eines Gemisches aus Wasser, Ameisensäure, Methanol und überschüssigem Methylformiat;
b) destillative Abtrennung des Methanols und des überschüssigen Methylformiats aus dem Gemisch aus Wasser, Ameisensäure, Methanol und überschüssigem Methylformiat unter Erhalt von wässriger Ameisensäure;
c) Extraktion der wässrigen Ameisensäure mit mindestens einem Ameisensäureester aus der Gruppe, die gebildet ist von Ethylenglycoldiformiat, Diethylenglycoldifonniat, 1,2-Propandioldiformiat, 2,3 Propandioldiformiat, Dipropylenglycoldiformiat, 2,3-Butandioldiformiat, 1,4-Butandioldiformiat, Benzylformiat, Cyclohexylformiat, 2-Phenylformiat, 2-Ethylhexylformiat, unter Erhalt eines Gemisches aus Ameisensäureester und Ameisensäure unter Destillationsbedingungen in einer Extraktivdestillationskolonne;
d) destillative Trennung von Ameisensäureester und Ameisensäure.

2. Verfahren nach Anspruch 1, **dadurch gekennzeichnet, dass** der Ameisensäureester Benzylformiat ist.

3. Verfahren nach Anspruch 1 oder 2, wobei der Ameisensäureester aus dem Alkohol und Ameisensäure in der Extraktivdestillationskolonne erzeugt wird.

4. Verfahren nach einem der Ansprüche 1 bis 3, wobei die Extraktivdestillationskolonne bei Umgebungsdruck betrieben wird.

5. Verfahren nach einem der Ansprüche 1 bis 4, wobei der Ameisensäureester und/oder der Alkohol am oder nahe dem Kopf der Extraktivdestillationskolonne aufgegeben wird.

6. Verfahren nach einem der Ansprüche 1 bis 5, wobei ein Phasenscheider am Kolonnenkopf der Extraktivdestillationskolonne vorgesehen ist.

7. Verfahren nach einem der Ansprüche 1 bis 6, wobei das Gemisch aus Ameisensäureester und Ameisensäure bei vermindertem Druck in einer Vakuumkolonne aufgetrennt wird.

8. Verfahren nach einem der Ansprüche 1 bis 7, wobei das Benzylformiat zwischen Extraktivdestillationskolonne und Vakuumkolonne im Kreislauf geführt wird.

## Claims

1. A process for the preparation of formic acid, comprising the following steps:
a) hydrolysis of methyl formate to give a mixture of water, formic acid, methanol and excess methyl formate;
b) removal of the methanol and excess methyl formate from the mixture of water, formic acid, methanol and excess methyl formate by distillation to give aqueous formic acid;
c) extraction of the aqueous formic acid with at least one formic ester selected from the group consisting of ethylene glycol diformate, diethylene glycol diformate, 1,2-propanediol diformate, 2,3-propanediol diformate, dipropylene glycol diformate, 2,3-butanediol diformate, 1,4-butanediol diformate, benzyl formate, cyclohexyl formate, 2-phenyl formate and 2-ethylhexyl formate, to give a mixture of ester formate and formic acid under distillation conditions in an extractive distillation column;
d) separation of formic ester and formic acid by distillation.

2. A process as claimed in claim 1, **characterized in that** the formic acid ester is benzyl formate.

3. A process as claimed in claim 1 or 2, where the formic ester is produced from the alcohol and formic acid in the extractive distillation column.

4. A process as claimed in one of claims 1 to 3, where the extractive distillation column is operated at ambient pressure.

5. A process as claimed in one of claims 1 to 4, where the formic ester and/or the alcohol are/is introduced at or close to the top of the extractive distillation column.

6. A process as claimed in one of claims 1 to 5, where a phase separator is provided at the top of the extractive distillation column.

7. A process as claimed in one of claims 1 to 6, where the mixture of formic ester and formic acid is separated under reduced pressure in a vacuum column.

8. A process as claimed in one of claims 1 to 7, where the benzyl formate is circulated between the extractive distillation column and the vacuum column.

## Revendications

1. Procédé de préparation d'acide formique, comprenant les étapes consistant à :
a) hydrolyser le formiate de méthyle, pour obtenir un mélange d'eau, d'acide formique, de méthanol et de formiate de méthyle en excès ;
b) séparer par distillation le méthanol et le formiate de méthyle en excès du mélange d'eau, d'acide formique, de méthanol et de formiate de méthyle en excès, pour obtenir l'acide formique aqueux ;
c) extraire l'acide formique aqueux avec au moins un ester de l'acide formique, choisi parmi le groupe qui est formé par le diformiate d'éthylèneglycol, le diformiate de diéthylèneglycol, le diformiate de 1,2-propanediol, le diformiate de 1,3-propanediol, le diformiate de dipropylèneglycol, le diformiate de 2,3-butanediol, le diformiate de 1,4-butanediol, le formiate de benzyle, le formiate de cyclohexyle, le formiate de 2-phényle, le formiate de 2-éthylhexyle, pour obtenir un mélange d'ester de l'acide formique et de l'acide formique dans des conditions de distillation, dans une colonne de distillation extractive ;
d) séparer par distillation l'ester de l'acide formique et l'acide formique.

2. Procédé selon la revendication 1, **caractérisé en ce que** l'ester de l'acide formique est le formiate de benzyle.

3. Procédé selon la revendication 1 ou 2, où l'ester de l'acide formique est produit à partir de l'alcool et de l'acide formique dans la colonne de distillation extractive.

4. Procédé selon l'une quelconque des revendications 1 à 3, où la colonne de distillation extractive fonctionne à pression ambiante.

5. Procédé selon l'une quelconque des revendications 1 à 4, où l'ester de l'acide formique et/ou l'alcool sont chargés à ou près de la tête de la colonne de distillation extractive.

6. Procédé selon l'une quelconque des revendications 1 à 5, où un séparateur de phase est prévu en tête de colonne de la colonne de distillation extractive.

7. Procédé selon l'une quelconque des revendications 1 à 6, où le mélange de l'ester de l'acide formique et de l'acide formique est séparé sous pression réduite, dans une colonne sous vide.

8. Procédé selon l'une quelconque des revendications 1 à 7, où le formiate de benzyle est recyclé entre la colonne de distillation extractive et la colonne sous vide.
